# EUROPEAN PATENT APPLICATION

(11) **EP 1 914 566 A2**
(43) Date of publication of application: **23.04.2008**
(21) Application number: 07019443.6
(22) Date of filing: 04.10.2007
(51) Int. Cl.: G01S 7/52

(54) **Ultrasound system and method for forming ultrasound images**

(30) Priority: 17.10.2006 KR 20060100935
(71) Applicant: MEDISON CO., LTD., Kangwon-do 250-870 (KR)
(72) Inventor: Kim, Jung Soo 3 FL, R & D Center, Medison Co. Ltd, Gangnam-gu Seoul 135-280 (KR)
(74) Representative: Schmid, Wolfgang

(57) **Abstract**

There is disclosed an ultrasound system for providing a color flow image. The system may include: a signal acquisition unit to obtain an ultrasound image signal of an object and a Doppler signal in a color box on said object, wherein said color box is set by a user; an input unit to receive color box setting information and region of interest (ROI) setting information; a processor to form a B-mode (Brightness-mode) image signal and a color flow image signal based on said ultrasound image signal and said Doppler signal, and form blood flow image information corresponding to said set ROI; and an output unit to display a B-mode image and a color flow image based on said B-mode image signal and said color flow image signal, and display said formed blood flow image information.

## Description

The present application claims priority from Korean Patent Application No. 10-2006-100935 filed on October 17, 2006, the entire subject matter of which is incorporated herein by reference.

### BACKGROUND

### 1. Field

The present invention generally relates to ultrasound image processing, and more particularly to a system and method for forming a color flow image for medical diagnosis purposes.

### 2. Background

An ultrasound system has become an important and popular diagnostic tool since it has a wide range of applications. Specifically, due to its non-invasive and nondestructive nature, the ultrasound system has been extensively used in the medical profession. Modem high-performance ultrasound systems and techniques are commonly used to produce two or three-dimensional diagnostic images of internal features of an object.

In order to transmit and receive ultrasound signals, the ultrasound system is generally provided with a probe including a wideband transducer. When the transducer is electrically stimulated, it produces ultrasound signals and transmits them into a human body. The ultrasound signals transmitted into the human body are reflected from borders between human tissues and then returned to the transducer. The returned ultrasound echo signals are converted into electric signals. Thereafter, ultrasound image data for imaging the tissues is produced by amplifying and signal-processing the echo signals.

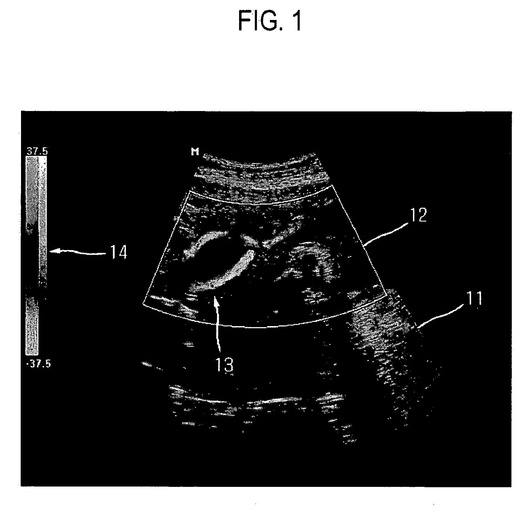

The ultrasound system may use the Doppler Effect to produce a color flow image, which represents the speed of a moving object. Fig. 1 is an exemplary picture that simultaneously displays a B-mode (Brightness-mode) image and a color flow image. When a user sets a color box 12 on the B-mode image 11 by using an input unit (e.g., track ball, mouse, keyboard, etc.), the ultrasound system forms and displays a color flow image 13 and a color map 14 based on the Doppler data corresponding to the color box 12. The color flow image 13 shows a red color for representing the blood flow, which moves forward to the transducer. Such an image also displays a blue color for representing the blood flow that moves backward from the transducer. The color map 14 represents the speed of the blood flow. The color positioned at a level above the zero baseline is red, while that positioned at a level below the zero baseline is blue. The color becomes darker as it is closer to the baseline. On the other hand, the color becomes brighter as it is farther from the baseline. Such changes in color represent the changes in the speed of the blood flow. A brighter color means that the speed of the blood flow is high.

However, the conventional ultrasound system does not provide blood flow image information (especially information on blood flow speed) corresponding to the color of a certain pixel in the color flow image. Therefore, a user cannot obtain the exact speed of the blood flow based only on the color of the pixel.

### BRIEF DESCRIPTION OF THE DRAWINGS

Arrangements and embodiments may be described in detail with reference to the following drawings in which like reference numerals refer to like elements and wherein:

Fig. 1 is an exemplary picture simultaneously displaying a B-mode image and a color flow image including a color map;

Fig. 2 is a block diagram of an ultrasound system according to one embodiment of the present invention;

Fig. 3 is an exemplary color map-velocity mapping table according to one embodiment of the present invention; and

Fig. 4 is a flow chart illustrating a process for forming an ultrasound image according to one embodiment of the present invention.

### DETAILED DESCRIPTION OF THE PRESENT INVENTION

A detailed description may be provided with reference to the accompanying drawings. One of ordinary skill in the art may realize that the following descriptions are illustrative only and are not in any way limiting. Other embodiments of the present invention may readily suggest themselves to such skilled persons having the benefit of this disclosure.

An ultrasound system of the present invention may include a signal acquisition unit, an input unit, a processor and an output unit. The signal acquisition unit may be configured to transmit an ultrasound signal to an object and receive the ultrasound signal reflected from the object to obtain an ultrasound image signal of the object. The signal acquisition unit may obtain a Doppler signal in a color box, which may be set by the user, on the object. The input unit may be configured to receive information on the color box setting and region of interest (ROI) setting from the user. The color box setting information may include the size and location of the color box. The ROI setting information may include the size and location of the ROI, which is set on the color flow image. The processor may be configured to form a B-mode (Brightness-mode) image signal and a color flow image signal based on the ultrasound image signal and the Doppler signal. The processor may further be configured to form blood flow image information corresponding to the ROI. The output unit may be configured to display a B-mode image and a color flow image based on the B-mode image signal and the color flow image signal. The output unit may also be configured to display the blood flow image information.

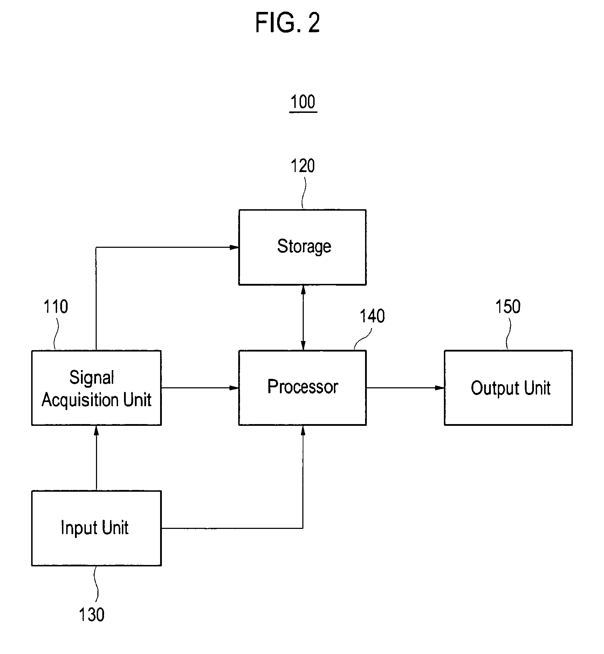

Additional detailed explanations on the embodiments of the present invention will be provided below with reference to Figs. 2 to 4.

As illustrated in Fig. 2, the ultrasound system 100 of the present invention may include a signal acquisition unit 110, a storage 120, an input unit 130, a processor 140 and an output unit 150.

To form an ultrasound image, the signal acquisition unit 110 may be configured to transmit an ultrasound signal to an object and receive the ultrasound signal reflected from the object to obtain an ultrasound image signal, which is used for forming a B-mode image of the object. The signal acquisition unit 110 may be configured to receive the color box setting information, which includes the size and location of the color box. As described above, the color box may be set on the B-mode image by the user. The signal acquisition unit 110 may also be configured to obtain a Doppler signal in the color box. The signal acquisition unit 110 may include a probe (not shown) for transmitting and/or receiving an ultrasound signal to/from an object through a plurality of transducers. It may also include a controller (not shown) for controlling the transmission/reception of ultrasound signals through the transducers. The controller may control the transmission/reception of ultrasound signals for obtaining the ultrasound image signal of the object and the Doppler signal in the color box.

The storage 120 may store the ultrasound image signal of the object and the Doppler signal in the color box, which is outputted from the signal acquisition unit 110. It may also store predetermined scale information, which includes the maximum and minimum velocities.

The input unit 130 may be configured to receive information on the color box setting and the ROI setting from the user. The color box setting information may include information on the size and location of the color box, which is set by the user on the B-mode image displayed on the output unit 150. The ROI setting information may include information on the size and location of the ROI, which represents a region in the color flow image for displaying the blood flow image information. The ROI may be set by the user on the color flow image, which is displayed on the output unit 150. Herein, the ROI may be composed of any one or combinations of point, line, cross section, etc. The input unit 130 may also be configured to receive select information from the user. The select information may include information on at least one image signal and Doppler signal among a plurality of ultrasound image signals and Doppler signals stored in the storage 120.

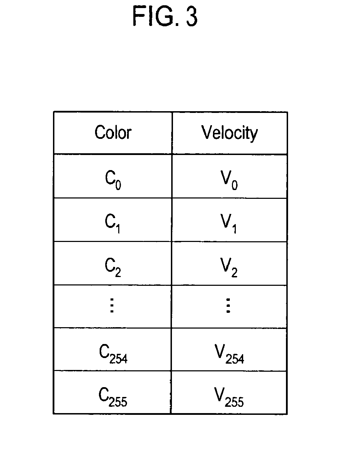

The processor 140 may be configured to form a B-mode image signal based on the ultrasound image signal of an object, which is inputted from the signal acquisition unit 110 or the storage 120. The processor 140 may also be configured to form a color flow image signal based on the Doppler signal inputted from the signal acquisition unit 110 or the storage 120, as well as the color box setting information inputted from the input unit 130. The processor 140 may be configured to form a table of velocities corresponding to the color map of the color flow image ("a color map-velocity mapping table") based on the predetermined scale information. According to one embodiment of the present invention, as illustrated in Fig. 3, the processor 140 may be configured to calculate velocities V₀ to V₂₅₅, each of which corresponds to each of the plurality of color indexes C₀ to C₂₅₅ in the color map, based on the predetermined scale information including the maximum and minimum velocities. Then, the processor 140 may be configured to form the color map-velocity mapping table based on the calculated velocities V₀ to V₂₅₅· Herein, V₀ is the minimum velocity and V₂₅₅ is the maximum velocity. The processor 140 may also be configured to temporarily store the formed color map-velocity mapping table in the storage 120. When an ROI is set on the color flow image through the input unit 130, the processor 140 may be configured to form the blood flow image information on the pixel(s) in the ROI. More specifically, the processor 140 may be configured to select at least one pixel in the ROI set on the color flow image, detect the color of the selected pixel, retrieve the velocity of the color index corresponding to the detected color from the color map-velocity mapping table and form the blood flow image information including the retrieved velocity. The blood flow image information may be displayed on the output unit 150 in the form of value(s) or a graph.

The output unit 150 may be configured to display a B-mode image and a color flow image based on the B-mode image signal and the color flow image signal, respectively, which are received from the processor 140. The output unit 150 may also be configured to display the blood flow image information, which is formed in the processor 140.

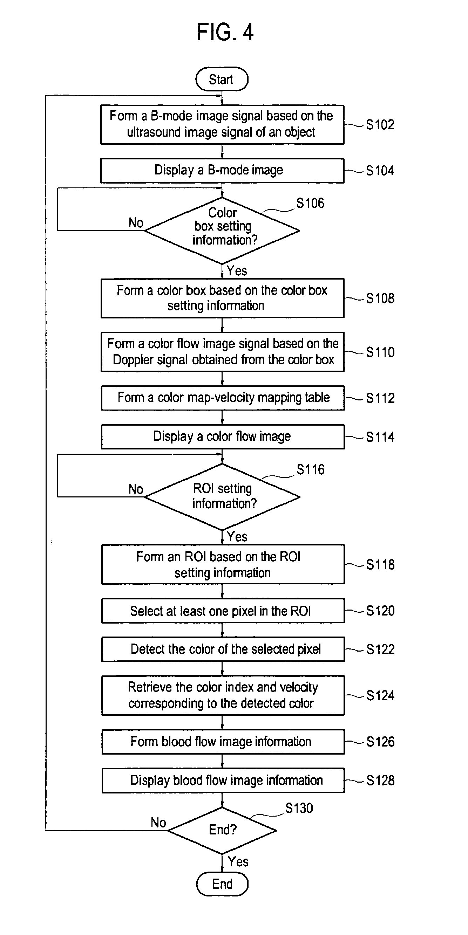

A process for forming an ultrasound image according to one embodiment of the present invention will be explained below with reference to Fig. 4.

As illustrated in Fig. 4, when an ultrasound image signal of an object is inputted from the signal acquisition unit 110 or the storage 120, the processor 140 forms a B-mode image signal based on the ultrasound image signal of the object at S102. The output unit 150 receives the B-mode image signal from the processor 140 to display a B-mode image at S104.

If color box setting information is inputted into the processor 140 through the input unit 130 at S106, then the processor 140 forms a color box based on the inputted color box setting information at S108. At S110, the processor 140 forms a color flow image signal based on the Doppler signal obtained from the color box. Then, the processor 140 forms a color map-velocity mapping table based on the predetermined scale information, which includes the maximum and minimum velocities, at S112. The output unit 150 receives the color flow image signal from the processor 140 to display a color flow image at S114.

At S116, the processor 140 determines whether ROI setting information is inputted from the user through the input unit 130. If it is determined that ROI setting information is not inputted, then the processor 140 waits until the ROI setting information is inputted. On the other hand, if it is determined that ROI setting information is inputted at S116, then the processor 140 forms an ROI based on the inputted ROI setting information at S118. Then, the processor 140 selects at least one pixel in the ROI at S120 and detects the color of the selected pixel at S122. The processor 140 retrieves the color index and the velocity corresponding to the detected color from the color map-velocity mapping table at S124. Then, the processor 140 forms the blood flow image information including the retrieved velocity at S126. The output unit 150 receives the blood flow image information from the processor 140 to display the received blood flow image information at S128.

Thereafter, the processor 140 determines whether the process for forming an ultrasound image according to one embodiment of the present invention has ended at S130. If it is determined that the process has not ended, then the process goes back to S102. Otherwise, the process executed in the ultrasound system 100 has ended.

According to the embodiments of the present invention, the blood flow image information on at least one pixel in the ROI is provided. Therefore, the user can obtain the exact velocity of the blood flow based on the blood flow image information.

According to one embodiment of the present invention, an ultrasound system may be provided to provide a color flow image. Such a system may include: a signal acquisition unit to obtain an ultrasound image signal of an object and a Doppler signal in a color box on said object, wherein said color box is set by a user; an input unit to receive color box setting information and ROI setting information; a processor to form a B-mode (Brightness-mode) image signal and a color flow image signal based on said ultrasound image signal and said Doppler signal, and form blood flow image information corresponding to said set ROI; and an output unit to display a B-mode image and a color flow image based on said B-mode image signal and said color flow image signal, and display said formed blood flow image information.

According to another embodiment of the present invention, a method may be provided to form an ultrasound image. Such a method may include the following steps: a) transmitting an ultrasound signal to an object and receiving an ultrasound signal reflected from said object to obtain an ultrasound image signal of said object; b) forming a B-mode (Brightness-mode) image based on said ultrasound image signal; c) displaying said formed B-mode image; d) receiving color box setting information, wherein said information includes size and location of a color box set on said B-mode image by a user; e) obtaining a Doppler signal in said color box based on said color box setting information; f) forming a color flow image based on said Doppler signal, wherein said color flow image includes a color map; g) displaying said formed color flow image; h) receiving ROI setting information, wherein said information includes size and location of an ROI set on said color flow image by a user; i) forming blood flow image information corresponding to said ROI based on said ROI setting information; and j) displaying said formed blood flow image information.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system for providing a color flow image, comprising:
a signal acquisition unit for obtaining an ultrasound image signal of an object and a Doppler signal in a color box on said object, wherein said color box is set by a user;
an input unit for receiving color box setting information and region of interest (ROI) setting information;
a processor for forming a B-mode (Brightness-mode) image signal and a color flow image signal based on said ultrasound image signal and said Doppler signal, the processor further being configured to form blood flow image information corresponding to said set ROI; and
an output unit for displaying a B-mode image and a color flow image based on said B-mode image signal and said color flow image signal, the output unit further being configured to display said formed blood flow image information.

2. The ultrasound system of Claim 1, wherein said blood flow image information includes information on blood flow speed.

3. The ultrasound system of Claim 2, wherein said processor comprises:
an image signal forming unit for forming said B-mode image signal and said color flow image signal based on said ultrasound image signal and said Doppler signal;
a color map-velocity mapping table forming unit for setting a plurality of color indexes based on a color map of said color flow image and setting velocities each of which corresponds to each of said plurality of color indexes, the color map-velocity mapping table forming unit further being configured to form a color map-velocity mapping table based on said plurality of color indexes and said velocities; and
a blood flow image information forming unit for selecting at least one pixel in said ROI and detecting color of said selected pixel, the blood flow image information forming unit further being configured to retrieve velocity corresponding to said detected color from said color map-velocity mapping table and form said blood flow image information.

4. The ultrasound system of Claim 3, wherein said color map-velocity mapping table forming unit calculates said velocities each of which corresponds to each of said plurality of color indexes based on predetermined scale information including maximum and minimum velocities.

5. A method of forming an ultrasound image, comprising.
a) transmitting an ultrasound signal to an object and receiving an ultrasound signal reflected from said object to obtain an ultrasound image signal of said object;
b) forming a B-mode (Brightness-mode) image based on said ultrasound image signal;
c) displaying said formed B-mode image;
d) receiving color box setting information, wherein said information includes a size and a location of a color box set on said B-mode image by a user;
e) obtaining a Doppler signal in said color box based on said color box setting information;
f) forming a color flow image based on said Doppler signal, wherein said color flow image includes a color map;
g) displaying said formed color flow image;
h) receiving region of interest (ROI) setting information, wherein said information includes a size and a location of an ROI set on said color flow image by a user;
i) forming blood flow image information corresponding to said ROI based on said ROI setting information; and
j) displaying said formed blood flow image information.

6. The method of Claim 5, wherein said blood flow image information includes information on blood flow speed.

7. The method of Claim 5, wherein said step i) comprises:
i-1) forming a color map-velocity mapping table based on said color map of said color flow image, wherein said color map-velocity mapping table includes a plurality of color indexes and velocities each of which corresponds to each of said plurality of color indexes; and
i-2) forming blood flow image information corresponding to said ROI based on said color map-velocity mapping table.

8. The method of Claim 7, wherein said step i-1) comprises:
i-11) setting a plurality of color indexes based on said color map of said color flow image;
i-12) setting velocities each of which corresponds to each of said plurality of color indexes based on predetermined scale information including maximum and minimum velocities; and
1-13) forming a color map-velocity mapping table based on said plurality of color indexes and said velocities.

9. The method of Claim 7, wherein said step i-2) comprises:
i-21) selecting at least one pixel in said ROI;
i-22) detecting color of said selected pixel;
i-23) retrieving velocity corresponding to said detected color from said color map-velocity mapping table; and
i-24) forming said blood flow image information including said retrieved velocity.
